(19) Europäisches Patentamt
European Patent Office
Office européen des brevets

(11) Publication number: **0 098 012**
**B1**

(12) **EUROPEAN PATENT SPECIFICATION**

(45) Date of publication of patent specification: **17.09.86**

(21) Application number: **83200915.3**

(22) Date of filing: **21.06.83**

(51) Int. Cl.⁴: **C 07 C 69/732,**
C 07 C 67/343, C 07 C 59/52,
C 07 C 59/56

(54) **Process for preparing (o-hydroxyaryl)-methanols.**

(30) Priority: **28.06.82 IT 2207682**
**21.03.83 IT 2017883**

(43) Date of publication of application:
**11.01.84 Bulletin 84/02**

(45) Publication of the grant of the patent:
**17.09.86 Bulletin 86/38**

(84) Designated Contracting States:
**AT BE CH DE FR GB IT LI LU NL SE**

(56) References cited:
**BULLETIN DE LA SOCIETE CHIMIQUE DE FRANCE, part II, September /October 1979, pages 583-591, Masson Distributeur, Paris, FR., J. MORVAN et al.: "Réaction du dioxosuccinate de méthyle avec les phénols. II. Synthèse et propriétés de quelques dérivés du dihydro-2,3 dihydroxy-2,3 benzo(b)furanne dicarboxylate-2,3 de méthyle"**

**CHEMICAL ABSTRACTS, vol. 91, no. 23, 3rd December 1979, page 615, no. 193005r, Columbus, Ohio, USA**

(73) Proprietor: **BLASCHIM S.p.A.**
**Via Vittor Pisani, 28**
**I-20124 Milano (IT)**
(73) Proprietor: **CONSIGLIO NAZIONALE DELLE RICERCHE**
**Piazzale Aldo Moro, 7**
**I-00185 Roma (IT)**

(72) Inventor: **Citterio, Attilio**
**Via Borgazzi 4**
**Monza (MI) (IT)**
Inventor: **Tinucci, Laura**
**Via Verdi, 24**
**San Giuliano Milanese (MI) (IT)**
Inventor: **Piccolo, Oreste**
**Via Cassa di Risparmi 31**
**Livorno (LI) (IT)**
Inventor: **Valoti, Ermanno**
**Piazza Leone XIIIo, 10**
**Dalmine (BO) (IT)**
Inventor: **Filippini, Lucio**
**Via Fratelli Cervi 27**
**Saronno (VA) (IT)**
Inventor: **Gandolfi, Marco**
**Via Garibaldi 44**
**Codogno (MI) (IT)**

**EP 0 098 012 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
CHEMICAL ABSTRACTS, vol. 93, no. 5, 4th August 1980, page 882, no. 46191m, Columbus, Ohio, USA

(74) Representative: Marchi, Massimo et al
c/o Marchi & Mittler s.r.l. Viale Lombardia 20
I-20131 Milano (IT)

**Description**

This invention relates to a process for preparing (o-hydroxyaryl)-methanols by reacting a phenol compound not substituted on the para position with a carbonyl compound in the presence of titanium tetrachloride and an aprotic diluent.

It is known that phenol compounds not substituted on the para position react in an acid medium with compounds of formula

$$R(OCO)_m—COY \qquad (III)$$

wherein

R is hydrogen, alkyl, substituted alkyl, cycloalkyl, aryl, or heterocyclic;

m is 0 or 1; and

Y is —COR, —$CR(OB)_2$, —COOR or —CN, where R has the above mentioned meaning and B is alkyl, arylalkyl, or both B radicals are an alkylidene radical which together with the group

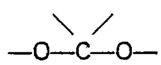

to which it is bound, forms an alicyclic system;

to give derivatives which are mono- and poly-substituted on the para position (E. Bannucci: Synthesis, (1973) 671—672; C. L. Parris et al: J.O.C. *27*, (1962) 455—60; Hofman, Schriesheim: Friedel-Crafts and related reactions, Vol. 2, New York, Olah Ed., Interscience Publishers) (1963/64), 597—640; Schnell, Krimm: Angew. Chemie, (Int. Ed.) *2*(1963) 373—379.

D. Molho et al. (Bull. Soc. Chim. Fr., (1956), 78—93) disclose the reaction of phenol compounds not substituted on the para position with a pyruvic acid, more particularly phenylpyruvic acid, in the presence of aluminium tetrachloride. According to these authors the reaction affords a complex mixture of many compounds among which no (o-hydroxyaryl)-methanol is present. J. Morvan et al. (Bull. Soc. Chim., (1979) part II, 583—591) disclose the reaction of phenol compounds not substituted on the para position with the methyl ester of dioxosuccinic acid in the presence of aluminium trichloride to afford derivatives of the dimethyl ester of 2,3-dihydro-2,3-dihydroxy-benzo-[b]-furan-2,3-dicarboxylic acid which by subsequent cleavage with potassium hydroxyde, give the corresponding (o-hydroxyaryl)mandelic acids. The dioxosuccinic acid and its esters are not embraced by the formula $R(OCO)_m—COY$ and their reaction with the phenol compounds does not produce directly an (o-hydroxyaryl)methanol.

Now it has been found that the compounds of formula R(OCO)—COY react with phenol compounds not substituted on the para position in the presence of titanium tetrachloride and an aprotic diluent to give (o-hydroxyaryl)-methanols according to the following scheme

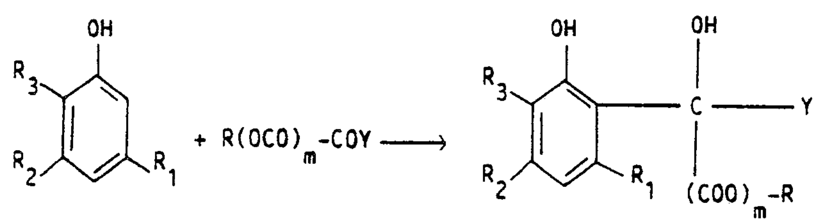

(II) (III) (I)

wherein

R is hydrogen, (1—6 C)-alkyl, (3—6 C)-cycloalkyl, (3—6 C)-cycloalkyl substituted by one or two alkyl groups each having from 1 to 4 C atoms, aryl, or phenyl-(1—3 C)-alkyl;

m is 0 or 1;

Y is —COR, —$CR(OB)_2$, —COOR or —CN, wherein R has the above mentioned meanings, and B is (1—6 C)-alkyl, or aryl(1—3 C)-alkyl, or both B radicals are an alkylidene radical which together with the group

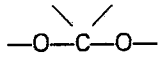

forms a 5—7 membered alicyclic system having from 3 to 9 C atoms; and

$R_1$, $R_2$ and $R_3$, the same or different, are hydrogen, (1—6 C)-alkyl, (3—6 C)-cycloalkyl, aryl, aryl-(1—3 C)alkyl, halo-aryl, alkoxy(1—3 C)-aryl, alkyl(1—6 C)-aryl, a heterocyclic ring having 5—7 members wherein one or two members are oxygen, sulphur or nitrogen, dialkyl(1—6 C)-amino, (2—10 C)-acylamino, (2—8 C)-alkyl- or arylimido, halogen, —O—A, —S—A, —OCOA where A is hydrogen, (1—6 C)-alkyl, phenyl-(1—3 C)alkyl, aryl optionally substituted by (1—6 C)-alkyl, (1—3 C)-alkoxy or by halogen, or $R_2$ and $R_3$ taken together are the groups necessary to form an aryl ring optionally substituted by (1—3 C)-alkoxy or halogen.

Particular embodiments of this invention are the preparations of compounds of formula I wherein

R is H, (1—3 C)-alkyl, (3—6 C)-cycloalkyl, aryl, or phenyl-(1—3 C)-alkyl;

m is 0 or 1;

Y is —COOAlkyl (1—3 C), —COOCycloalkyl or —COOH; and

$R_1$, $R_2$ and $R_3$, the same or different, are hydrogen, (1—6 C)-alkyl, (3—6 C)-cycloalkyl, phenyl optionally substituted by one or two halogen, aryl-(1—3 C)alkyl, hydroxy, halogen, (1—6 C)-alkoxy, phenoxy optionally substituted by one or two halogen, (1—6 C)-alkanoyloxy, (1—6 C)-alkylthio, thiophenoxy optionally substituted by one or two halogen, or $R_2$ and $R_3$ taken together are a butanedienylidene chain which completes a naphthyl ring optionally substituted by (1—3 C)-alkoxy or halogen.

Specific embodiments of this invention are the preparations of compounds of formula I wherein

R is H, methyl, cyclohexyl, phenyl, or benzyl;

m is 0 or 1;

Y is —COOH, —$COOC_2H_5$, —$COOCH_3$, —COOmenthyl; and

$R_1$, $R_2$ and $R_3$, the same or different, are hydrogen, methyl, isobutyl, cyclohexyl, phenyl, 2,4-difluorophenyl, benzyl, hydroxy, chlorine, bromine, methoxy, phenoxy, acetoxy, methylthio, or $R_2$ and $R_3$ taken together are a butanedienylidene chain which completes a naphthyl ring optionally substituted by methoxy or chlorine.

Preferred aprotic diluents according to this invention are methylene chloride, dichloroethane, benzene, nitromethane, and nitrotoluene.

Temperature and reaction time are largely dependent on the reactivity of the phenol compound (II) to the substitutions of electrophilic type so that the reaction may also occur in only few minutes at −80°/20°C, whereas it takes several days (at 15°/20°C) when the phenol compound (II) is less reactive to the electrophilic substitutions.

Addition of the various reactants does not have to follow a fixed sequence; a possible way consists of dissolving 1 mole of compound (II) and 1 mole of compound (III) in the diluent, bringing the solution to the desired temperature and adding 1 mole of titanium tetrachloride while stirring.

Alternatively 1 mole of compound (II) and 1 mole of titanium tetrachloride may be added to the diluent, then the temperature is adjusted and 1 mole of compound (III) is added. Before adding compound (III) it may be useful to reduce the acidity of the medium by removing all or part of the haloacid formed.

Peculiar features of this process are the specificity of the substitution on the ortho position and the high yields which are almost quantitative for the phenol compounds (II), which are most reactive to electrophilic substitution.

When $R_1$ (3-position) is different from hydrogen and both $R_2$ and $R_3$ are hydrogen, compound (III) enters selectively on position 6. When $R_3$ is hydrogen and both $R_1$ and $R_2$ are different from hydrogen, mixtures of 2- and 6-substituted isomers are formed. The quantities of the isomers thus obtained differ in accordance with the nature of $R_1$ and $R_2$ as well as with the reaction conditions.

It is therefore possible to prepare the methanols (I) in high yields in a very simple and economical way.

The (o-hydroxyaryl)-methanols (I) of formula

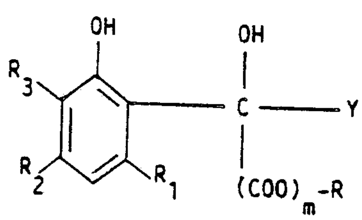

(I)

wherein

R, Y, $R_1$, $R_2$, $R_3$ and m have the above mentioned meanings are new except when m is 0 R is H, Y is —COOH, and

$R_3$ is chlorine and both $R_1$ and $R_2$ are hydrogen; or

$R_2$ and $R_3$ are methyl and $R_1$ is hydrogen.

They may be used for preparing benzofuranones, (Italian Patent Application No. 24817 A/82 filed on Dec. 7, 1982), benzofurans, o-hydroxy-arylalkanoic acids, o-hydroxyarylmalonic acid, arylalkanoic acids, o-acyl-phenols or derivatives thereof according to well known techniques.

The invention is further described and exemplified by the detailed examples which follow but are not intended to limit the scope of the invention.

### Example 1

1-(2'-hydroxyphenyl)-1-methyl-1-ethoxycarbonyl-1-methanol

Phenol (4.98 g; 53 mmol) and ethyl pyruvate (6.15 g; 53 mmol) were dissolved in methylene chloride (30 ml); the solution thus obtained was cooled to 0°C and titanium tetrachloride (10.05 g; 53 mmol) was added while stirring. The reaction mixture was stirred at 0°C for 3 hours and at room temperature for 5 hours more.

The reaction mixture was treated with water; after separation of the organic layer, the aqueous layer was extracted several times with methylene chloride.

The organic extracts were collected and dried over sodium sulphate and the solvent was removed under reduced pressure. The crude residue (9.40 g) (chemical purity, 90%; NMR analysis) was purified by chromatography on silica gel (eluent, n-hexane/diethyl ether 9/1); 6.8 g of chemically pure 1-(2'-hydroxyphenyl)-1-methyl-1-ethoxy-carbonyl-1-methanol (viscous oil) were thus obtained. Yield, 80%.

$C_{11}H_{14}O_4$

| | | |
|---|---|---|
| Anal. Calcd: | C, 62.84; | H, 6.71 |
| Found: | 62.70; | 6.68 |

In an analogous manner but using:

— 3-methoxyphenol instead of phenol, 1-(4'-methoxy-2'hydroxyphenyl)-1-methyl-1-ethoxycarbonyl-1-methanol was obtained. Yield, 93%.

$C_{12}H_{16}O_5$

| | | |
|---|---|---|
| Anal. Calcd: | C, 59.99; | H, 6.71 |
| Found: | 60.05; | 6.65 |

— 3-methylphenol instead of phenol, 1-(4'-methyl-2'hydroxyphenyl)-1-methyl-1-ethoxycarbonyl-1-methanol was obtained. Yield, 75%.

$C_{12}H_{16}O_4$

| | | |
|---|---|---|
| Anal. Calcd: | C, 64.27; | H, 7.19 |
| Found: | 64.21; | 7.22 |

Example 2

1-(2'-hydroxyphenyl)-1-methyl-1-carboxy-1-methanol

Phenol (0.94 g; 10 mmol) was dissolved in methylene chloride (15 ml); the solution thus obtained was cooled to 0°C and titanium tetrachloride (1.89 g; 10 mmol) was added.

Into this mixture was dropped under stirring a solution of pyruvic acid (0.88 g; 10 mmol) in methylene chloride (7 ml). After the addition was over the reaction mixture was stirred for one hour and a half at 0°C.

Afterwards the reaction mixture was treated with 10% hydrochloric acid; the organic layer was separated and the aqueous layer was extracted several times with methylene chloride.

The pooled organic extracts were dried over sodium sulphate and the solvent was removed under reduced pressure. The crude residue (1.69 g) was purified by crystallization from diethyl ether; a white crystalline solid was thus obtained, m.p. 137—140°C. Yield, 75%

$C_9H_{10}O_4$

| | | |
|---|---|---|
| Anal. Calcd: | C, 59.33; | H, 5.53 |
| Found: | 59.20; | 5.61 |

In an analogous manner but using:

— 3-methoxy-phenol instead of phenol, 1-(4'-methoxy-2'-hydroxyphenyl)-1-methyl-1-carboxy-1-methanol a white solid melting at 111—113°C (diethyl ether) was obtained. Yield 70%

$C_{10}H_{12}O_5$

| | | |
|---|---|---|
| Anal. Calcd: | C, 56.60; | H, 5.70 |
| Found: | 56.65; | 5.59 |

— ethyl pyruvate instead of pyruvic acid, 1-(2'-hydroxyphenyl)-1-methyl-1-ethoxycarbonyl-1-methanol was obtained. Yield, 92%.

— 3-methyl-phenol instead of phenol and ethyl pyruvate instead of pyruvic acid, 1-(4'-methyl-2'-hydroxyphenyl)-1-methyl-1-ethoxycarbonyl-methanol was obtained. Yield, 90%.

— resorcin instead of phenol and ethyl pyruvate instead of pyruvic acid, 1-(2',4'-dihydroxyphenyl)-1-methyl-1-ethoxycarbonyl-1-methanol was obtained. Yield, 80%.

$C_{11}H_{14}O_5$

| | | |
|---|---|---|
| Anal. Calcd: | C, 58.40; | H, 6.24 |
| Found: | 58.49; | 6.30 |

— 3-isobutyl-phenol instead of phenol and ethyl pyruvate instead of pyruvic acid, 1-(4'-isobutyl-2'-hydroxyphenyl)-1-methyl-1-ethoxycarbonyl-1-methanol was obtained. yield, 83%.

$C_{15}H_{22}O_4$

| | | |
|---|---|---|
| Anal. Calcd: | C, 67.64; | H, 8.33 |
| Found: | 67.59; | 8.35 |

— 2-methyl-phenol instead of phenol and ethyl pyruvate instead of pyruvic acid, 1-(3'-methyl-2'-hydroxyphenyl)-1-ethoxycarbonyl-1-methanol was obtained. Yield, 92%.

$C_{12}H_{16}O_4$

| | | |
|---|---|---|
| Anal. Calcd: | C, 64.27; | H, 7.19 |
| Found: | 64.35; | 7.16 |

— 3-acetoxy-phenol instead of phenol and ethyl pyruvate instead of pyruvic acid, 1-(4'-acetoxy-2'-hydroxyphenyl)-1-methyl-1-ethoxycarbonyl-1-methanol was obtained. Yield, 79%.

$C_{13}H_{16}O_6$

| | | |
|---|---|---|
| Anal. Calcd: | C, 58.20; | H, 6.01 |
| Found: | 58.26; | 6.05 |

— 1-naphthol instead of phenol and ethyl cyclohexyl-glyoxylate instead of pyruvic acid, 1-[2'-(1'-hydroxy)-naphthyl]-1-cyclohexyl-1-ethoxycarbonyl-1-methanol was obtained. Yield, 75%.

$C_{20}H_{24}O_4$

| | | |
|---|---|---|
| Anal. Calcd: | C, 73.14; | H, 7.37 |
| Found: | 73.25; | 7.35 |

— 3-hydroxy-5-methyl-phenol instead of phenol and ethyl pyruvate instead of pyruvic acid, 1-(6'-methyl-2',4'-dihydroxyphenyl)-1-methyl-1-ethoxycarbonyl-1-methanol was obtained. Yield. 40%.

$C_{12}H_{16}O_5$

| | | |
|---|---|---|
| Anal. Calcd: | C, 59.99; | H, 6.71 |
| Found: | 60.02; | 6.65 |

Example 3

1-(4'-bromo-2'-hydroxyphenyl)-1-methyl-1-ethoxycarbonyl-1-methanol

A solution of 3-bromo-phenol (9.3 g; 53.7 mmol) was dropped into a mixture of titanium tetrachloride (10.2 g; 53.9 mmol) in methylene chloride (50 ml).

The mixture thus obtained was warmed to 40°C and a solution of ethyl pyruvate (6.24 g; 53.7 mmol) in methylene chloride (50 ml) was added dropwise.

After stirring the reaction mixture for 5 hours at the boiling temperature, a further aliquot of ethyl pyruvate (4 g; 34.4 mmol) was added. After 34 hours the reaction mixture was cooled to 0°C and poured into an aqueous solution of dilute hydrochloric acid; the organic layer was separated and the aqueous layer was extracted with methylene chloride.

The pooled organic extracts were washed with water and dried over sodium sulphate.

After evaporation of the solvent, the crude product was purified by chromatography on silica gel (eluent, n-hexane/ethylacetate 7:3). Yield, 75%.

$C_{11}H_{13}O_4Br$

| | | | |
|---|---|---|---|
| Anal. Calcd: | C, 45.69; | H, 4.53; | Br, 27.64 |
| Found: | 45.60; | 4.61; | 27.29 |

— 2-chloro-phenol instead of phenol, 1-(3'-chloro-2'-hydroxyphenyl)-1-methyl-1-ethoxycarbonyl-1-methanol was obtained. Yield, 35%.

$C_{11}H_{13}O_4Cl$

| | | | |
|---|---|---|---|
| Anal. Calcd: | C, 53.99; | H, 5.36; | Cl, 14.49 |
| Found: | 53.90; | 5.42; | 14.55 |

— 3-phthalimido-phenol instead of phenol and 50% excess of titanium tetrachloride, 1-(4'-phthalimido-2'-hydroxy-phenyl)-1-methyl-1-ethoxycarbonyl-1-methanol was obtained. Yield, 45%.

$C_{19}H_{17}NO_6$

| | | | |
|---|---|---|---|
| Anal. Calcd: | C, 64.22; | H, 4.82; | N, 3.94 |
| Found: | 64.20; | 4.85 | 4.02 |

Example 4

1-[2'-(6'-methoxy-1'-hydroxy)-naphthyl]-1-methyl-1-ethoxycarbonyl-1-methanol

Titanium tetrachloride (17.97 g; 94.8 mmol) was added to a solution of 6-methoxy-1-naphthol (15 g; 86 mmol) in methylene chloride (300 ml).

The solution was cooled to −50°C and a solution of ethylpyruvate (11 g; 94.7 mmol) in methylene chloride (300 ml) was added while keeping the reaction mixture below −45°C.

After two hours the mixture was hydrolyzed and worked up as described in the previous examples. After purification via chromatography on silica gel by using n-hexane/ethyl acetate 7:3 as eluent, 21.2 g of chemically pure title compound (viscous oil) were obtained. Yield, 85%.

$C_{16}H_{18}O_5$

| | | |
|---|---|---|
| Anal. Calcd: | C, 66.19; | H, 6.25 |
| Found: | 66.22; | 6.33 |

In an analogous manner but using:

— 1-naphthol instead of 6-methoxy-1-naphthol, 1-[2'-(1'hydroxy)-naphthyl]-1-methyl-1-ethoxycarbonyl-1-methanol was obtained. Yield, 98%.

$C_{15}H_{16}O_4$

| | | |
|---|---|---|
| Anal. Calcd: | C, 69.21; | H, 6.20 |
| Found: | 69.31; | 6.18 |

— 1-naphthol instead of 6-methoxy-1-naphthol and methyl phenyl glyoxylate instead of ethylpyruvate, 1-[2'-(1'-hydroxy)-naphthyl]-1-phenyl-1-methoxycarbonyl-1-methanol was obtained. Yield, 78%.

$C_{19}H_{16}O_4$

| | | |
|---|---|---|
| Anal. Calcd: | C, 74.01; | H, 5.23 |
| Found: | 74.12; | 5.25 |

— 1-naphthol instead of 6-methoxy-1-naphthol and ethyl glyoxylate instead of ethyl pyruvate, 1-[2'-(1'-hydroxynaphthyl]-1-ethoxycarbonyl-1-methanol was obtained. Yield, 70%.

$C_{14}H_{14}O_4$

| | | |
|---|---|---|
| Anal. Calcd: | C, 68.28; | H, 5.73 |
| Found: | 68.22; | 5.80 |

— 1-naphthol instead of 6-methoxy-1-naphthol and ethyl phenyl pyruvate instead of ethyl piruvate, 1-[2'-(1'-hydroxy)-naphthyl]-1-benzyl-1-ethoxycarbonyl-1-methanol was obtained. Yield, 70%.

$C_{21}H_{20}O_4$

| | | |
|---|---|---|
| Anal. Calcd: | C, 74.98; | H, 5.99 |
| Found: | 74.82; | 6.02 |

Example 5

(−) 1-[2'-(1'-hydroxy)-naphthyl]-1-methyl-1-carboxy-1-methanol.

Menthyl pyruvate was prepared according to K. Matsumoto and K. Harada (J. Org. Chem., *31*, 1956—62, 1966) but using Amberliest 15 (registered trade mark) instead of p-toluenesulfonic acid. Yield, 85—95%; b.p. 105—107°C (3 mm Hg); $[\alpha]^{D}_{20} = -84.77$ (c, 1.887; absolute ethanol). Titanium tetrachloride (2.24 ml; 20.4 mmol) was added to a solution of 1-naphthol (2.94 g; 20.4 mmol) in methylene chloride (100 ml) cooled to −40°C. After 10 minutes a solution of menthyl pyruvate (4.941 g; 20.4 mmol) in methylene chloride (10 ml) was added while keeping the temperature at −40°C.

After 10 minutes 10% hydrochloric acid (30 ml) was added and the temperature was allowed to raise to 25°C.

With the usual working up technique, 9.9 g of crude product were obtained $[\alpha]^{D}_{20} = -169.74$ (c, 2.00; absolute ethanol). Purity (T.L.C.) of the crude product was higher than 95%. HPLC assay on RP 8 and RP 18 columns indicates the absence of significant amounts of diasteroisomeric forms (purity: 98%; stereoisomers: less than 2%).

2 g of the ester thus obtained were hydrolyzed in ethanol (15 ml) with 30% NaOH (2 ml) at room temperature. After 3 hours water (5 ml) was added and the reaction mixture was extracted with diethyl ether (2 × 5 ml). The aqueous layer was made acid at 0°C with 10% HCl and extracted with diethyl ether.

The ethereal extracts were pooled and dried over anhydrous sodium sulfate. The solid residue was crystallized from diethyl ether/petroleum ether (1:1); (−) 1-[2'-(1'-hydroxy)-naphthyl]-1-methyl-1-carboxy-1-methanol was thus obtained with the chemical purity over 95%; m.p. 103—4°C; $[\alpha]^{D}_{20} = -74.17$ (c, 2.00; absolute ethanol).

## Example 6

1-(2'-hydroxyphenyl)-1,1-dicarbethoxy-1-methanol

Phenol (4.7 g; 50 mmol) was dissolved in 1,2-dichloroethane (100 ml); the solution thus obtained was cooled to 0°C and titanium tetrachloride (5.5 ml; 50 mmol) was added. The reaction mixture was maintained under a slow nitrogen stream for 10 minutes.

A solution of diethyloxomalonate (8.7 g; 50 mmol) in 1,2-di-chloroethane (20 ml) was dropped into this mixture while stirring. After the addition was complete the mixture was stirred for two hours at 0°C and for 30 minutes at room temperature.

The cooled reaction mixture was then treated with 10% hydrochloric acid (35 ml) and kept under stirring for 10 minutes; the organic layer was separated and the aqueous layer was extracted with 1,2-dichloroethane (20 ml).

The pooled organic extracts were dried over sodium sulphate and the solvent was removed under reduced pressure. The crude residue (12.5 g) was purified by chromatography on silica gel (eluent, hexane/ethyl ether, 8:3). 10.2 g (Yield, 76%) of pure product (T.L.C. and N.M.R. assays) were thus obtained.

$C_{13}H_{16}O_6$

| | | |
|---|---|---|
| Anal. Calcd: | C, 58.20; | H, 6.01 |
| Found: | 58.29; | 6.00 |

In an analogous manner but replacing:

— phenol with 1-naphthol, 1,2-dichloroethane with dichloromethane and carrying out the reaction at −50°C for two hours and a half 1-[2'-(1'-hydroxy)-naphthyl]-1,1-dicarbethoxy-1-methanol melting at 59°—60°C was obtained. Yield, 80%.

$C_{17}H_{18}O_6$

| | | |
|---|---|---|
| Anal. Calcd: | C, 64.14; | H, 5.70 |
| Found: | 64.21; | 5.68 |

— phenol with m-cresol, 1,2-dichloroethane with dichloromethane and carrying out the reaction at −40°C for one hour, 1-(2'-hydroxy-4'-methylphenyl)-1,1-dicarbethoxy-1-methanol was obtained. Yield, 85%.

$C_{14}H_{18}O_6$

| | | |
|---|---|---|
| Anal. Calcd: | C, 59.56; | H, 6.43 |
| Found: | 59.49; | 6.51 |

— phenol with 3-isobutyl-phenol and, 1,2-dichloroethane with dichloromethane, 1-(2'-hydroxy-4'-isobutylphenyl)-1,1-dicarbethoxyl-methanol melting at 63—64°C (pentane) was obtained. Yield, 85%.

$C_{17}H_{24}O_6$

| | | |
|---|---|---|
| Anal. Calcd: | C, 62.95; | H, 7.46 |
| Found: | 62.89; | 7.50 |

— phenol with 6-methoxy-naphthol and, 1,2-dichloroethane with dichloromethane and carrying out the

reaction at −40°C, 1-[2′-(1′-hydroxy-6′-methoxy)-naphthyl]-1,1-dicarbethoxy-1-methanol melting at 73°C was obtained. Yield, 70%.

$C_{18}H_{20}O_7$

| | | |
|---|---|---|
| Anal. Calcd: | C, 62.06; | H, 5.79 |
| Found: | 62.10; | 5.75 |

## Claims

1. A process for preparing compounds of formula

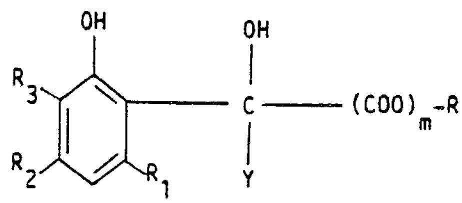

(I)

where

$R_1$, $R_2$ and $R_3$, the same or different, are hydrogen, (1—6 C)-alkyl, (3—6 C)-cycloalkyl, aryl, aryl-(1—3 C)alkyl, halo-aryl, alkoxy(1—3 C)-aryl, alkyl(1—6 C)-aryl, a heterocyclic ring having 5—7 members wherein one or two members are oxygen, sulphur or nitrogen, dialkyl(1—6 C)-amino, (2—10 C)-acylamino, (2—8 C)-alkyl- or arylimido, halogen, —O—A, —S—A or —OCOA where A is hydrogen, (1—6 C)-alkyl, phenyl-(1—3 C)alkyl, aryl optionally substituted by (1—6 C)-alkyl, (1—3 C)-alkoxy or by halogen, or $R_2$ and $R_3$ taken together are the groups necessary to form an aryl ring optionally substituted by (1—3 C)-alkoxy or halogen;

m is 0 or 1;

R is hydrogen, (1—6 C)-alkyl, (3—6 C)-cycloalkyl, (3—6 C)-cycloalkyl substituted by one or two alkyl groups each having from 1 to 4 C atoms, aryl, or phenyl-(1—3 C)-alkyl; and

Y is —COR, $—CR(OB)_2$, —COOR or —CN, where R has the above mentioned meanings, and B is (1—6 C)-alkyl or aryl-(1—3 C)-alkyl, or both B radicals are an alkylidene radical which together with the group

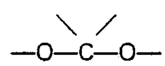

forms a 5—7 membered alicyclic system having from 3 to 9 C atoms; characterized in that a phenol compound of formula

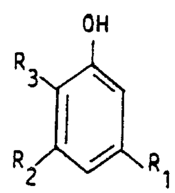

(II)

where

$R_1$, $R_2$ and $R_3$ have the above meanings, is reacted in the presence of titanium tetrachloride and an aprotic diluent with a compound of formula

$$R(OCO)_m—COY \qquad (III)$$

where

R, Y and m have the above mentioned meanings.

2. Process according to claim 1, characterized in that the aprotic diluent is methylene chloride, dichloroethane, benzene, nitrobenzene or nitromethane.

**Patentansprüche**

1. Verfahren zur Herstellung der Verbindungen der Formel

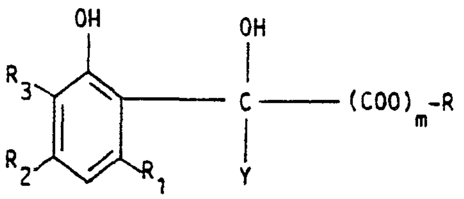

(I)

worin

$R_1$, $R_2$ und $R_3$, die gleich oder verschieden sein können, für Wasserstoff, $C_1$—$C_6$-Alkyl, $C_3$—$C_6$-Cycloalkyl, Aryl, Aryl-$C_1$—$C_3$alkyl, Halo-aryl, $C_1$—$C_3$-Alkoxy-aryl, $C_1$—$C_6$-Alkyl-aryl, einen heterocyclischen Ring mit 5 bis 7 Gliedern, wobei ein oder zwei Glieder Sauerstoff, Schwefel oder Stickstoff sind, $C_1$—$C_6$-Dialkylamino, $C_2$—$C_{10}$-Acylamino, $C_2$—$C_8$-Alkyl- oder Arylimido, Halogen, —O—A, —S—A, oder —OCOA stehen, wobei A für Wasserstoff, $C_1$—$C_6$-Alkyl, Phenyl-$C_1$—$C_3$-Alkyl oder Aryl (gewünschtenfalls substituiert durch $C_1$—$C_6$-Alkyl, $C_1$—$C_3$ Alkoxy oder Halogen) steht, oder $R_2$ und $R_3$ zusammen diejenigen Gruppen bedeuten, die zur Bildung eines Arylrings erforderlich sind, der gewünschtenfalls durch $C_1$—$C_3$ Alkoxy oder Halogen substituiert ist;

m für 0 oder 1 steht;

R für Wasserstoff, $C_1$—$C_6$-Alkyl, $C_3$—$C_6$-Cycloalkyl, durch ein oder zwei Alkylgruppen mit jeweils 1 bis 4 C-Atomen substituiertes $C_2$—$C_6$-Cycloalkyl, Aryl oder Phenyl-$C_1$—$C_3$-alkyl steht; und

Y für —COR, —CR(OB)$_2$, —COOR oder —CN steht, wobei R die obigen Bedeutungen besitzt und B für $C_1$—$C_6$-Alkyl oder Aryl-$C_1$—$C_3$-alkyl steht, oder beide Reste B einen Alkylidenrest bilden, der zusammen mit der Gruppe

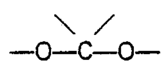

ein 5 bis 7-gliedriges alicyclisches System mit 3 bis 9 C-Atomen bilden; dadurch gekennzeichnet, daß man eine Phenolverbindung der Formel

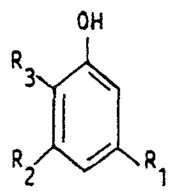

(II)

worin

$R_1$, $R_2$ und $R_3$ die oben angegebenen Bedeutungen besitzen, in Gegenwart von Titantetrachlorid und einem aprotischen Lösungsmittel mit einer Verbindung der Formel

$$R(OCO)_m—COY \qquad (III)$$

worin

R, Y und m die oben angegebenen Bedeutungen besitzen, umsetzt.

2. Verfahren nach Anspruch 1 dadurch gekennzeichnet, daß man als aprotisches Lösungsmittel Methylenchlorid, Dichlorethan, Benzol, Nitrobenzol oder Nitromethan einsetzt.

**Revendications**

1. Procédé pour la préparation de composés de formule

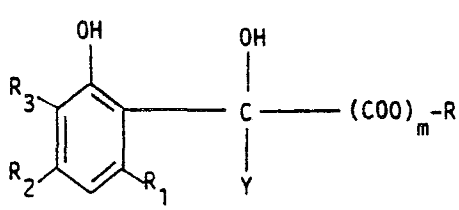

(I)

dans laquelle

$R_1$, $R_2$ et $R_3$, semblables ou différents, sont chacun un atome d'hydrogène, un groupement (1—6 C)-alkyle, (3—6 C)-cycloalkyle, aryle, aryl-(1—3 C)-alkyle, halogéno-aryle, alcoxy(1—3 C)-aryle, alkyl-(1—6 C)-aryle, un noyau hétérocyclique à 5—7 termes, dans lequel un ou deux termes sont des atomes d'oxygène, de soufre ou d'azote, un groupement dialkyl-(1—6 C)-amino, (2—10 C)-acylamino, (2—8 C)-alkyl- ou arylimido, un atome d'halogène, un groupement —O—A, —S—A, ou —OCOA, dans lequel A est un atome d'hydrogène, un radical (1—6 C)-alkyl, phényl-(1—3 C)-alkyle, aryle éventuellement substitué par un radical (1—6 C)-alkyle, (1—3 C)-alcoxy ou par un atome d'halogène, ou $R_2$ et $R_3$ forment ensemble les groupes nécessaires pour constituer un noyau aryle éventuellement substitué par un radical (1—3 C)-alcoxy ou un atome d'halogène;

m est égal à 0 ou 1;

R est un atome d'hydrogène, un groupement (1—6 C)-alkyle, (3—6 C)-cycloalkyle, (3—6 C)-cycloalkyle substitué par un ou deux groupes alkyle contenant chacun 1 à 4 atomes de C, aryle ou phényle-(1—3 C)-alkyle; et

Y est un groupement —COR, —CR(OB)$_2$, —COOR ou —CN, R ayant les significations données ci-dessus et B étant un radical (1—6 C)-alkyle, aryl-(1—3 C)-alkyle ou les deux radicaux B étant un radical alkylidène formant, avec le groupement

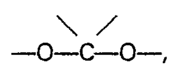

un système alicyclique à 5—7 termes contenant 3 à 9 atomes de C, caractérisé en ce qu'un composé phénolique de formule

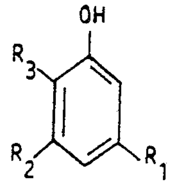

(II)

dans laquelle

$R_1$, $R_2$ et $R_3$ ont les significations données ci-dessus, est mis à réagir, en présence de tétrachlorure de titane et d'un diluant aprotique, avec un composé de formule

$$R(OCO)_m—COY \qquad (III)$$

dans laquelle

R, Y et m ont les significations données ci-dessus.

2. Procédé selon la revendication 1, caractérisé en ce que le diluant aprotique est le chlorure de méthylène, le dichloroéthane, le benzène, le nitrobenzène ou le nitrométhane.